# EUROPEAN PATENT SPECIFICATION

(11) **EP 0 929 503 B1**
(45) Date of publication and mention of the grant of the patent: **17.10.2001**
(21) Application number: 97909974.4
(22) Date of filing: 03.10.1997
(51) Int. Cl.: C07C 29/10, C07C 31/20

(54) **A METHOD FOR PRODUCING GLYCOLS**
VERFAHREN ZUR HERSTELLUNG VON GLYKOLEN
METHODE DE FABRICATION DE GLYCOLS

(30) Priority: 04.10.1996 US 26659 P
(43) Date of publication of application: 21.07.1999
(73) Proprietor: THE DOW CHEMICAL COMPANY, Midland, Michigan 48674 (US)
(72) Inventor: STRICKLER, Gary, R., Midland, MI 48642 (US); WOMACK, Joseph, L., Oakland, CA 94618 (US)
(74) Representative: Raynor, John
(86) International application number: US9717936
(87) International publication number: WO9814419

(56) References cited:
- US-A- 4 165 440

## Description

This invention relates to a method for producing glycols. Such a method is particularly useful for preparing alkylene glycols, such as ethylene glycols.

Commercial processes for the preparation of alkylene glycols, for example, ethylene glycol, propylene glycol and butylene glycol, involve the liquid phase hydration of the corresponding epoxide in the presence of a large molar excess of water *(see, for example*, Kirk-Othmer, *Encyclopedia of Chemical Technology,* Vol. 11, Third Edition, page 929 (1980)). The hydrolysis reaction is typically conducted at moderate temperatures (for example 100 to 200 °C) with water being provided to the reaction zone in excess of 15 to 20 moles per mole of epoxide. The primary by-products of the hydrolysis reaction are di-, tri-, and higher glycols. The formation of the di- and polyglycols is believed to be primarily due to the reaction of the epoxide with alkylene glycols. As epoxides are generally more reactive with glycols than they are with water, the large excess of water is employed in order to favor the reaction with water and thereby obtain a commercially attractive selectivity to the monoglycol product. However, even in light of the large excess of water, a typical commercially practiced method for making ethylene glycol has a selectivity to EG of between 80 to 90 weight percent (wt%), a selectivity to diethylene glycol (DEG) of between 9 to 15 wt%, and a selectivity to triethylene glycol (TEG) of between 1 to 5 wt%.

Since the glycols must be recovered from the hydrolysis reaction mixtures, the large excess of water can result in an energy intensive procedure. Typically, the water is removed by evaporation to leave a glycol containing residue which may be purified by distillation. Therefore, it would be beneficial, at least from the standpoint of energy efficiency, to reduce the amount of water employed while maintaining, or enhancing, selectivity toward the monoglycol product.

Therefore, the method of this invention is a synergistic combination of the processes of evaporation, absorption, and reaction, combined to enhance reaction yields for reactions of epoxides with compounds containing hydroxyl functional groups. Such method comprises making glycol in an evaporation column reactor as follows: (a) feeding a hydroxyl compound into the reactor; (b) feeding an epoxide into the reactor such that the epoxide compound is contacted with hydroxyl compound in a reaction zone under conditions such that substantially all of the epoxide reacts to form a product comprising a glycol which is dissolved in liquid hydroxyl compound; (c) removing the mixture of liquid hydroxyl compound and dissolved glycol away from the reaction zone; and (d) heating the mixture of liquid hydroxyl compound and dissolved glycol such that at least some of the hydroxyl compound is evaporated away from the glycol; and (e) condensing and refluxing the evaporated hydroxyl compound and combining it with the hydroxyl compound of Step (a). In one aspect of the invention, the epoxide feed described in Step (b) enters the reactor through at least two distinct ports within the reactor. In another aspect of this invention, the method is synergistically conducted in at least two sequential effects (for example evaporation columns) which together form a multiple effect evaporation column reactor, wherein the unevaporated hydroxyl compound, and glycol dissolved therein, in Step (d) is transferred from the first effect to a subsequent effect of the evaporation column reactor, at a point below the reaction zone of the subsequent effect, such that it becomes the feed of the subsequent effect's Step (a).

The method of this invention is useful to increase the yield of glycols from epoxides and hydroxyl compounds as compared to typical processes known in the art. The required amount of hydroxyl compound is also minimized as compared to typical processes known in the art. Selectivity to the desired number of glycol alkylations may also be increased. For example, when the desired composition is mono-alkylene glycol, by removing the mono-alkylene glycol as more specifically set forth herein, its selectivity is greatly increased with respect to di-, tri-, and higher glycols. Finally, the multiple effect reactive evaporation aspect of the invention provides desirable energy efficiency compared to a typical single effect reactor system.

Figure 1 illustrates a single effect evaporation column reactor having multiple epoxide feed ports.

Figure 2 illustrates a dual effect evaporation column reactor having single epoxide feed ports in each effect.

Figure 3 illustrates a triple effect evaporation column reactor having multiple epoxide feed ports in each effect.

In each of the figures the following reference numbers apply: **1** is the Hydroxyl Compound Feed; **2** is the Epoxide Compound Feed; **3** is the Distillate; **4** is the Enriching Section of the Reaction Zone; **5** is the Reaction Zone; **6** is the Stripping Section of the Reaction Zone; **7** is the Bottoms Product Draw; **8** is the Reboiler Zone; **9** is the Condensate Return; **10** is the Overhead Purge; **11** is the Product Stream; **12** is the Condensing and Refluxing Zone; and **13** is the Catalyst Bed of the Reaction Zone.

The method of this invention comprises the synergistic combination of the steps of evaporation, absorption, and reaction to enhance glycol yields from reactions of epoxides with compounds containing hydroxyl functional groups. Such a method utilizes either a single effect evaporation column reactor having more than one feed of epoxide into the reactor, or it utilizes a multiple effect evaporation column reactor which, optionally, has more than one feed of epoxide into the reactor.

More specifically, the "single effect" evaporation column reactor comprises: a reaction zone for reacting the epoxide and hydroxyl compound; a reboiler zone which is located below the reaction zone; a condensing and refluxing zone which is located above the reaction zone; at least one hydroxyl feed port which is, preferably, located above or within the reaction zone; and at least two epoxide feed ports which are located above the reboiler zone. Although such zones will more specifically be defined hereinafter, in a generic sense such zones may be described as follows: the reboiler zone contains a reboiler and its function is to heat and evaporate; the reaction zone is the zone in which the desired reactions occur; and the condensing and reflux zone is where evaporated compositions may be condensed and refluxed as liquid back into the reactor. For purposes of this invention, an "effect" means that the reaction zone, reboiler zone, and condensing and reflux zone are all in fluid communication with each other such that the evaporation, reaction, and absorption of the method of this invention occurs.

The "multiple effect" evaporation column reactor comprises: the above "single effect" evaporation column reactor plus at least one subsequent effect in series which is in fluid communication with the first effect. The "fluid communication" is such that at least one liquid hydroxyl feed port of the subsequent effect is located below its reaction zone and such that any excess latent heat and/or heat of reaction released from the first effect's condensing and reflux zone is transferred to the reboiler zone of the subsequent effect. This "transferring" from one effect to a subsequent effect in series may be continued for any desired number of times, with the final transfer being for the purpose of product recovery by transferring the unevaporated hydroxyl compound, and glycol dissolved therein, out of the multiple effect evaporation column reactor. It is also possible to configure the multiple effect reactor for "fluid communication" in such a way that the excess latent heat and/or heat of reaction is transferred from one effect to a subsequent effect in an opposite direction from the direction in which the hydroxyl compound is fed.

The first step of the method comprises feeding a hydroxyl compound into the reactor. The hydroxyl compound may comprise any compound containing a hydroxyl functional group which is capable of reacting with an epoxide moiety to produce a glycol or glycol ether. The hydroxyl compound should have a boiling point which is higher than that of the epoxide moiety and lower than that of any glycol products. A preferred hydroxyl compound is water, but other hydroxyl compounds such as alcohols (for example methanol, ethanol, propanol, and butanol) or glycols may also be useful. For example, if the desired product is a mono-alkylene glycol, the preferred hydroxyl compound is water; if the desired product is a higher-alkylene glycol (for example di- or tri-alkylene glycol), the preferred hydroxyl compound is the respective mono-alkylene glycol; and if the desired product is a glycol ether, the preferred hydroxyl compound is an alcohol. The hydroxyl compound is provided in an amount which is in a stoichiometric excess of that required for forming a desired glycol from reaction with epoxide. For example, when using water as the hydroxyl compound, a preferred molar feed ratio of hydroxyl compound to epoxide is between a lower limit of 1.05, preferably 1.2, and more preferably 1.5, to an upper limit of 20, preferably 10, and more preferably 5. Those of skill in the art will recognize that this ratio will vary depending upon the hydroxyl and epoxide compounds employed.

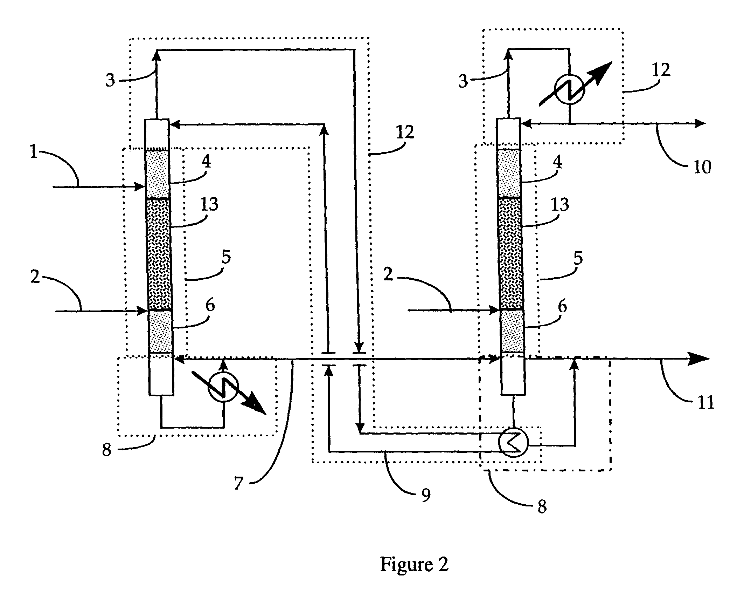

The hydroxyl compound may be fed into the reactor at any point within each effect. Generally, because it is advantageous to have liquid hydroxyl compound flowing downward into the reaction zone, the hydroxyl compound is preferably fed as liquid into the first effect of the reactor at a point above or within the reaction zone. This is especially preferred in a single effect evaporation column reactor and for the first effect of a multiple effect reactor. In such a single effect, as depicted in "Figure 2", the Hydroxyl Compound Feed **1** is supplied through a feed port in the Enriching Section **4** of the Reaction Zone **5** and allowed to flow further into the Reaction Zone **5** via gravitational forces. The liquid may also be combined with liquid hydroxyl compound being generated from the effect's condensing and reflux zone. In contrast to the single effect reactor, the subsequent effects in the multiple effect reactor may have liquid hydroxyl compound (typically containing glycol product) transferred to a point below the reaction zone, wherein the liquid hydroxyl compound is evaporated, condensed, and refluxed back down through the reaction zone, providing a feed of hydroxyl compound into the reaction zone.

The second step of the method comprises feeding an epoxide into the reactor such that the epoxide compound is contacted with the hydroxyl compound in the reaction zone under conditions such that substantially all of the epoxide reacts to form a product comprising a glycol. Those of skill in the art will recognize that once the hydroxyl compound is fed into the reactor, the hydroxyl compound the hydroxyl compound will be present in both gas and liquid phases depending upon temperature and pressure within the reactor. For example, within the reaction zone, the hydroxyl compound is present in a state of dynamic equilibrium wherein it is in both liquid and gaseous phases. Whether the hydroxyl compound is in a liquid or a gaseous state is not important as long as substantially all of the epoxide is reacted to form the desired product comprising a glycol under the conditions provided in the evaporation column reactor.

The epoxide may be any alkylene oxide as long as its reaction with a hydroxyl compound yields the desired glycol. Preferably, the epoxide is either ethylene oxide, propylene oxide, or butylene oxide. It is important that conditions are provided such that substantially all of the epoxide reacts to form a product comprising a glycol. By "substantially" it is meant that greater than at least 99% of the epoxide feed is reacted. It is believed that if the reaction of the epoxide is not substantially complete, epoxide will escape to the condenser and the condensing temperature will be lowered or it will escape to the reboiler zone and react with the glycol product. In order to further ensure that epoxide does not escape out of the reaction zone, it is preferred to include a stripping section in between the reaction zone and any point to which product and/or hydroxyl compound is transferred. For example, within the reaction zone, at an interface directly adjacent to the reboiler zone, a "stripping section" may be included in order to inhibit transfer of epoxide into the reboiler zone by providing for fractionation and distillation of epoxide out of the liquid phase. Within the reaction zone, at an interface directly adjacent to the condensing and reflux zone, a similar "enriching section" may be included in order to strip undesirable components (for example, glycol products and/or unreacted epoxide), resulting in an enrichment (that is, concentration) of the hydroxyl compound before its entry into the condensing and refluxing zone. The stripping section and enriching section typically consist of packing materials or trays, as are known by those of skill in the art, wherein such materials are substantially unreactive to the reactants and products which are present in the reactor under the conditions and components for which the reactor is operated. Generally, it is desirable that such packings provide high surface area. For example, inert packings such as glass and ceramic, balls, irregular pieces, sheets, tubes, rings (for example, Raschig rings), saddles (for example, Berl saddles and INTALOX™ Saddles), and FLEXIPAK™ Packing may all be useful.

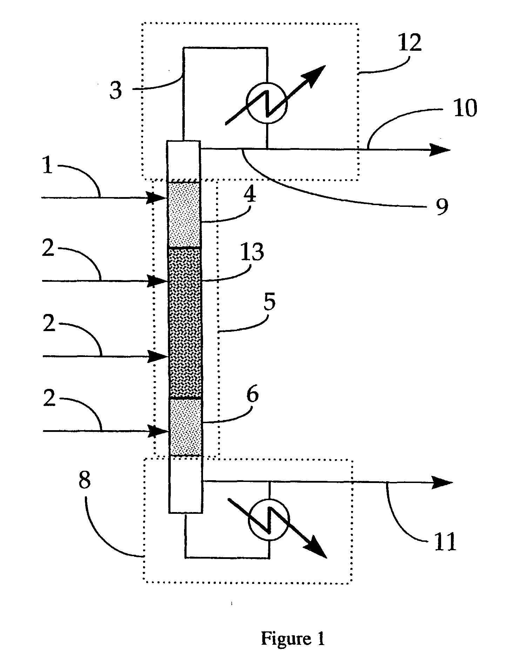

It is particularly advantageous in the method of this invention to split the epoxide feed so that it enters the reactor through at least two distinct ports within the reactor. For example, Figures 1, 2, and 3 each depict the use of three Epoxide Compound Feeds **2** in at least one effect. Although the total quantity of epoxide is generally equivalent to as if provided in a single feed stream, it has been discovered that separating the feed into more than one feed typically results in a higher selectivity to the desired glycol. If only a single feed of epoxide is used, the reaction zone behaves as a well mixed zone and the concentration of glycol which competes with water to react with the epoxide is constant near the epoxide feed port. This results in a lower monoglycol yields (as compared to multiple epoxide feeds) unless uneconomical excesses of hydroxyl compound are evaporated up through the reaction zone. If, however, the epoxide is split into multiple injection ports at different points along the reaction zone, the glycol product concentrates as it flows down the column. It is most preferred that the epoxide feed ports are spaced apart from each other such that substantially all of each feed port's epoxide compound has reacted in the vicinity of its feed port and is not transferred into the stream of epoxide entering at any other epoxide compound feed port. At similar boil-up ratios, the concentration reaches a level similar to the single feed system near the bottom of the reaction zone. However, since much of the epoxide is fed and reacts in a zone that has a lower concentration of glycol present, the selectivity increases from the bottom to the top of the reaction zone. Therefore, the average selectivity of a multi-epoxide feed system, which takes the whole column into account, is higher for similar boil-up and feed ratios than in a single epoxide feed system.

In one embodiment of this invention, the reaction zone comprises a catalyst bed. The catalyst bed may comprise any material capable of catalyzing the desired reaction in the evaporation column reactor reaction zone. It should be of such a nature as to allow a vapor flow of hydroxyl compound through the bed, yet provide a sufficient surface area for catalytic contact. The catalytic material can be a solid, liquid, or gas, as long as the catalytic material is not transferred into the reboiler zone. For example, the catalytic material could be a liquid or gas phase component such that its boiling point keeps it out of the reboiler zone. Examples include moieties of ammonia, hydrochloric acid, alkyl amines and carbon dioxide. Preferably, the catalytic material utilized in the catalyst bed reaction zone is a solid which is insoluble in either the reactants or the glycol products and is mechanically stable under the conditions in the process. For example, it may be a solid acid catalyst or a solid base catalyst or others such as catalytic metals and their oxides or halides suitable for a multitude of catalytic reactions and heterogeneous with the reaction or other components in the system. Depending upon the catalytic material utilized (for example, solid), the catalytic material may also serve an additional role in that the material may act as a stripping site encouraging the fractionation of glycol product from the hydroxyl compound. As described previously, the reaction zone also may contain distinct stripping/enriching sections for inhibiting the flow of epoxide to the reboiler or glycol products into the condensing and refluxing zone.

Representative examples of the numerous acid catalysts that may be useful in the hydration of alkylene oxides include the following: fluorinated alkyl sulfonic acid ion exchange resins (U.S. Pat. No. 4,165,440); carboxylic acids and halogen acids (U.S. Pat. No. 4,112,054); strong acid cation exchange resins (U.S. Pat. No. 4,107,221); aliphatic mono- and/or polycarboxylic acids (U.S. Pat. No. 3,933,923); cationic exchange resins (U.S. Pat. No. 3,062,889); acidic zeolites (U.S. Pat. No. 3,028,434); sulfur dioxide (U.S. Pat. No. 2,807,651); trihalogen acetic acids (U.S. Pat. No. 2,472,417); and copper-promoted aluminum phosphate (U.S. Pat. No. 4,014,945). Specific examples of desirable heterogeneous catalysts are aluminosilicate zeolites, amorphous aluminosilicates, and acid form ion exchange resins. A preferred catalyst is a perfluorosulfonic acid resin entrapped within and dispersed throughout a carrier of metal oxide. Preferably, the metal oxide is selected from the group consisting essentially of silica, alumina, titania, germania, zirconia, alumino-silicate, zirconyl-silicate, chromic oxide and iron oxide. Such types of catalysts are described in U.S. Patent No. 4,731,263 and PCT Publication No. WO 95/19222.

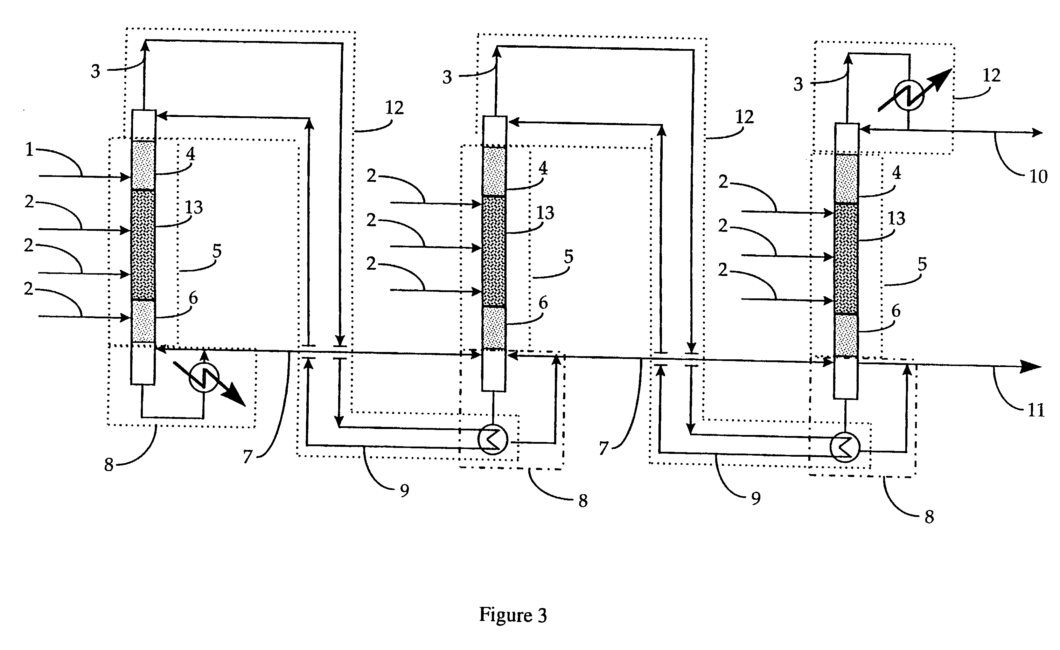

In one preferred embodiment of this invention, a multiple effect evaporation column reactor is utilized wherein the reaction zone of each subsequent effect contains a catalyst of higher catalytic activity than the previous effect's reaction zone. This is particularly advantageous because it is observed that. in one embodiment of this invention, reactivity 10 a desired monoglycol is highest in the first effect and progressively less in subsequent effects due to a typically progressive decrease in temperature in each subsequent effect. In order to increase the reactivity in the subsequent columns to maintain equivalent productivity, catalyst beds are provided in sufficient activity to provide an acceptable selectivity to the desired glycols. To illustrate, in Figure **3** the first column's Reaction Zone **5** might have no catalytic material present in the Reaction Zone **5**. but the second column might have a catalyst bed, and the final column's Reaction Zone **5** might have a catalyst bed of higher catalytic activity than the second column.

In addition, a graded catalytic bed may be used as an alternative to using multiple epoxide feeds (described above). In a "graded catalytic bed" catalyst of higher activity/reactivity is positioned more towards the top of the reaction zone and that of lesser activity towards the bottom of the reaction zone. The epoxide feed port is then located towards the bottom of the reaction zone. Once the epoxide is fed into the bottom of the reaction zone, the increased catalyst reactivity towards the top of the reaction zone can therefore compensate for the typical decrease in epoxide concentration at greater distances from the epoxide feed port. This enables the reaction zone to give substantially equal reactivity across its height and produce a reasonably constant amount of glycol. The glycol may then be concentrated and carried down and out of the reaction zone toward the reboiler zone.

The next step of the method comprises removing the mixture of liquid hydroxyl compound and dissolved glycol away from the reaction zone. By "dissolved glycol" it is meant that the glycol product is a component which is generally in mixture, solution, or contained within any unreacted liquid hydroxyl compound. In one embodiment of the invention, such removal may be attained merely by allowing the liquid hydroxyl compound to trickle down out of the reaction zone via the forces of gravity. Once the mixture is removed from the reaction zone, the glycols are allowed to concentrate in the reboiler zone, resulting in very low concentrations of glycols in the reaction zone. This maximizes the hydroxyl compound to product glycol ratio in the reaction zone and provides an environment for maximum selectivity to the desired product glycol. The product glycols, often in mixture with hydroxyl compound, may then be removed from the bottoms in order to recover the product and inhibit any further reaction of the glycol product (for example, reaction of mono-glycol product to higher glycols).

The mixture of liquid hydroxyl compound and dissolved glycol may be heated such that at least some of the hydroxyl compound is evaporated away from the glycol. Such heating may take place in the reboiler zone. When the hydroxyl compound evaporates, it is transferred up through the evaporation column through the reaction zone as vapor, and is eventually condensed and refluxed back to the reaction zone, typically under the influence of gravity, as liquid hydroxyl compound. When the hydroxyl compound is evaporated and transferred to and through the reaction zone, it is also useful for carrying epoxide compound up to and into the reaction zone. The reboiler zone is a heat source for the effect and typically comprises a volume of space below the reaction zone wherein epoxide compound is not present and includes a heat exchanger, a receptacle for containing liquids as they flow down the effect, and a circulation system. Heat exchangers are well understood by those of skill in the art and typically comprise a "hot side" (often referred to as "shell side") for transferring fluid of higher temperature, and a "cold side" (often referred to as "tube side") for transferring fluid of lower temperature, wherein the sides are such that there is no exchange of compositional matter between either of the sides, only heat. Therefore, the heat exchanger is useful for absorbing heat through the "hot side" from another effect's condensing and refluxing zone and transferring the heat into the "cold side" of the heat exchanger. In addition, the reboiler zone must be equipped for transferring liquids *(see, for example,* Figures 2 and 3 "Bottoms Product Draw" **7**) out of the effect, and preferably for the reboiler zones of subsequent effects to be equipped so that liquids (for example, the previous effect's "Bottoms Product Draw" **7**) may also be transferred into the reboiler zone. The circulation system of the reboiler zone is useful for this purpose and also for circulating the liquid hydroxyl compound through the cold side of the heat exchanger and back into the receptacle. An example of a circulation system is either a thermal siphon or a recirculating pump.

Depending upon the evaporation column reactor setup utilized (for example, single or multiple effect), the condensing and refluxing zone may comprise a traditional condensor as understood by those of skill in the art, or it may comprise a pair of transfer lines which are located between the reaction zone of one effect and the hot side of the heat exchanger of another effect of the multiple effect evaporation column reactor. Such transfer lines may simply consist of, for example, a piping network which circulates distillate (for example, evaporated hydroxyl compound) from the top of one effect (that is, above the reaction zone), through a distillate transfer line, to the heat exchanger of a subsequent effect, through the hot side of the heat exchanger, and returns condensate (for example, liquid hydroxyl compound) to the previous effect through a condensate transfer line. For example, in Figure's 2 and 3, Distillate **3** (that is, comprising evaporated hydroxyl compound) exits the top of the first effect, is circulated via the transfer line to a hot side of a heat exchanger in the Reboiler zone **8** of the second effect, and liquid hydroxyl compound is returned as "Condensate Return" **9** via another transfer line. The cold side of the heat exchanger in the second effect's reboiler zone captures the excess latent heat plus the heat of reaction from the first effect and allows the evaporated hydroxyl compound to condense and be returned to the first effect as liquid. By condensing in such a way, the excess latent heat and heat of reaction from the previous evaporation column may be utilized beneficially in the subsequent effect's reboiler zone. Typically, in such a multiple effect setup, the final effect may utilize a traditional condensor positioned at the top of the last effect, above the reaction zone, for condensing and refluxing the evaporated hydroxyl compound.

In light of the disclosure herein, those of skill in the art will recognize that when the condensing and refluxing zone is located between two separate effects it is not critical as to which effects are linked in this manner. For example, in one preferred embodiment, the heat is transferred to the same subsequent effect for which the unevaporated hydroxyl compound, and glycol dissolved therein (for example, the "Bottoms Product Draw" **7**, of Figure 3), such that the heat from the previous effect's distillate and the unevaporated hydroxyl compound move in a co-current direction *(see, for example*, Figure 3). In another embodiment, the heat from the previous effect's distillate is transferred to a different effect than the unevaporated hydroxyl compound, and glycol dissolved therein, such that the heat and the unevaporated hydroxyl compound move in a counter-current direction.

Once the hydroxyl compound is condensed, it may either be used exclusively as the liquid hydroxyl feed stream, or it may be combined with a new feed of hydroxyl compound. For example, in Figure 3, the Condensate Return **9** is combined with the Hydroxyl Compound Feed **1** of the first effect for reaction in the first effect's reaction zone with the Epoxide Compound **2**. However, in the second effect of Figure 3, there is no separate liquid hydroxyl feed above the reaction zone and therefore the Condensate Return **9** is not combined with a new feed of hydroxyl compound. The only hydroxyl compound for which the condensate is combined in the second effect is the evaporated hydroxyl compound which is being produced in the reboiler zone. In light of the disclosure herein, however, those of skill in the art will recognize that a fresh feed of hydroxyl compound could also be added above, or within, the reaction zone of the subsequent effects if so desired.

When a multiple effect evaporation reactor is utilized, unevaporated hydroxyl compound, and glycol product dissolved therein, is transferred to a subsequent evaporation column effect at a point below the reaction zone of the subsequent effect such that it becomes the hydroxyl compound feed of the subsequent effect. Since the hydroxyl compound being transferred contains glycol product, it is important that this hydroxyl feed is fed to a point below the subsequent effect's reaction zone to minimize its contact with epoxide compound that will be fed to the effect. Otherwise, the glycol product may further react to form higher glycols. Such a transferring may be continued for any desired number of times with the final transfer being for the purpose of product recovery by transferring the unevaporated hydroxyl compound, and glycol dissolved therein, out of the multiple effect evaporation column reactor. In order to evaporate the "unevaporated hydroxyl compound" once it has been transferred to the subsequent effect's reboiler zone, it is preferred that each subsequent effect of the multiple effect evaporation column reactor be maintained at a lower pressure than the pressure of the previous effect of the multiple effect evaporation column reactor. This maintenance of pressure in each effect may be accomplished by any means known by those of skill in the art such as an overheads purge on each effect that allows the pressure to be maintained at the desired pressure. It is also possible to evaporate the "unevaporated hydroxyl compound" in the subsequent effect by maintaining the temperature of each subsequent effect's reboiler zones at progressively higher temperatures.

It has been discovered that a synergistic effect results when such a multiple effect reactor system is utilized. In the manufacture of ethylene glycol from ethylene oxide and water, it is preferred to use a train of three effects. One result of such a multiple effect reactor system is that the latent heat and heat of reaction of the evaporated hydroxyl compound may be used over in multiple effects in order to provide energy efficiency in the reactor system. The success of the evaporation column reactor system may be attributed to several factors. For example, because the reaction is occurring concurrently with evaporation, the initial reaction product, glycol, is removed from the reaction zone nearly as quickly as it is formed. This removal of the glycol minimizes decomposition or further reactions to undesired byproducts. Further, because the hydroxyl compound is boiling, the temperature of the reaction is controlled by the boiling point of the hydroxyl compound at the system pressure. The heat of reaction simply creates more boil up which benefits reaction selectivity, but no increase in temperature.

The invention will be further clarified by a consideration of the following examples, which are intended to be purely exemplary of the use of the invention.

### Examples

For each of the following examples. a continuous reaction system was constructed of PYREX™. It consisted of a 1000 milliliter (mL), three-neck, round-bottom flask for a reboiler. The reboiler was heated by a heating mantle. To the reboiler, a 36 inch (91 cm) long by one inch (2.5 cm) diameter column was attached to the middle neck. The column was packed with eight inches (20 cm) of inert ¼ inch (6 mm) Berl saddles to form a stripping section in the reaction zone, and 16 inches of perfluorosulfonic acid resin catalyst above the stripping section. The catalyst was prepared by depositing perfluorosulfonic acid polymer onto ¼ inch diameter (6 mm) hollow cylinders of silicon carbide according to the teachings of U.S. Patent No. 4,731,263 to form a solid acid catalyst with 15 wt% perfluorosulfonic acid. About four inches (10 cm) of ¼ inch (6 mm) Berl saddles were then added to the top of the catalyst within the column to act as an enriching section. A one sixteenth inch (1.6 mm) feed port was added to the column ten inches (25 cm) above the reboiler and just below the top of the stripping section Berl saddle packing. To the top of the column, a condenser was attached. The condenser was cooled by a chilled coolant which was continuously circulated through it at 4 °C. Condensate fell by gravity directly onto the top of the reaction zone. The reboiler was operated at a pressure of about 2.7 Kpa (gauge) and achieved a temperature of 100 °C.

In each of the examples, the reaction products were analyzed by gas chromatography and quantified for ethylene glycol, diethylene and higher glycols, and water.

### EXAMPLE 1

To the reboiler described above, 651.45 grams (g) of water was added. The water was heated to boiling and allowed to evaporate up the column through the inert packing and catalyst and up into the condenser. The condenser was allowed to operate at total reflux, and after a steady stream of condensate began returning to the column, the ethylene oxide (EO) feed pump was turned on. The feed rate of EO was 10 g/hour. The boil up of water was determined to be 240 g/hour. The conversion of EO was determined to be 35% and the selectivity to ethylene glycol was 94%.

### EXAMPLE 2

To the reboiler described above, 547.5 g of water was added. The water was heated to boiling and allowed to evaporate up the column through the inert packing and catalyst and up into the condenser. The condenser was allowed to operate at total reflux, and after a steady stream of condensate began returning to the column, the EO feed pump was turned on. The feed rate of EO was 10 g/hour. The boil up of water was determined to be 180 g/hour. The conversion of EO was determined to be 41% and the selectivity to ethylene glycol was 93%.

### EXAMPLE 3

To the catalyst section in the reaction zone in the column described above, an additional 6 inches (15 cm) of the catalyst was added which provided a catalyst section in the reaction zone that was 21 inches (53 cm) high. To the reboiler described above, 651.45 g of water was added. The water was heated to boiling and allowed to evaporate up the column through the inert packing and catalyst and up into the condenser. The condenser was allowed to operate at total reflux, and after a steady stream of condensate began returning to the column, the EO feed pump was turned on. The feed rate of EO was 14.8 g/hour. The boil up of water was determined to be 300 g/hour. The conversion of EO was determined to be 56% and the selectivity to ethylene glycol was 93%.

### EXAMPLE 4 (multiple-effect evaporation column reactor)

As an example of a triple-effect evaporation column reactor having single epoxide feed ports in each effect, two more columns may be added in series to the column described for Examples 1-3, above, wherein the column described above is utilized as the third column of a triple effect reactor. Each column is identical to the column described above except that the top of the first column is connected to a transfer line for circulating distillate from the top of the first column around a heat exchanger of the second column and back up into the first column as condensate return. A similar transfer line is connected between the second and the third column. All the columns are connected in series such that the bottoms product draw from the first column is transferred to the second column, and the bottoms product draw from the second column is transferred to the third column. Each column is equipped with an overhead purge for controlling each columns pressure. The first column's reboiler is operated at a gauge pressure of between 0.5 MPa to 2.0 MPa and a temperature of between 140 °C to 200 °C. The second column's reboiler is operated at a pressure of between 0.2 MPa to 1.0 MPa and a temperature of between 120 °C to 160 °C. The third column's reboiler is operated at a pressure of between 0.05 MPa to 0.2 MPa and a temperature of between 80 °C to 120 °C.

To the reboiler described above of the first column, between 250 to 750 g of water is added. The water is heated to boiling and allowed to evaporate up the column through the inert packing and catalyst and up into and through the transfer line. After a steady stream of condensate begins to return to the column, the EO feed pump is turned on. The feed rate of EO is between 10 g/hour and 30 g/hour. As water and glycol product begins to accumulate in the reboiler of the first effect the unevaporated water and glycol product is drawn out by means of a transfer pump or pressure letdown valve and transferred to the reboiler of the second column. A similar process is conducted for the second column, as with the first, and the unevaporated water and glycol product from the second effect is transferred to the third effect. As water is evaporated up the third column it reacts with the EO feed and water and glycol product begins to accumulate in the reboiler of the third effect. Unreacted water vapor is condensed in the condensor and returned to the reaction zone as liquid for further reactions with the EO feed. In such a triple effect evaporation reactor, it is calculated that 1.6 pounds of steam will be required per pound of ethylene glycol produced at a boilup weight ratio of 8:1 (water to ethylene oxide).

### EXAMPLE 5 (multiple epoxide feed ports)

As an example of a single-effect evaporation column reactor having multiple epoxide feed ports, a manufacturing-scale simulation was conducted based upon measured kinetics and a reactor diameter of 16.1 feet (4.91 meters) having a total of 30 sieve trays, with Tray 30 being the reboiler. The effect of the multiple feed ports was examined by conducting computer-generated runs with the number of epoxide feed ports varying from 1 up to 8. The basic design of the set up was as set forth in "Figure 1", described herein, but no catalytic material was utilized in the reaction zone. The total rate of epoxide feed, irrespective of the number of feed ports, was kept constant at 50,000 pounds (22,679.6 kilograms) of EO per hour. The EO feed was kept equally distributed amongst the EO feed ports being utilized ("active ports"). The feed rate to inactive ports was zero. The boil-up to EO feed ratio was held constant at 10 pounds (4.5 kilograms) of boil-up to 1 pound (0.4 kilograms) of total EO feed. To illustrate TABLE 1, "Run 8" shows a total number of feed ports to he 8. These feed ports arc located at Trays 5, 8, 11, 14, 17, 20, 23, and 26. In "Run 8", 80,000 pounds/hour (36,287 kg/hr) of water was added as the hydroxyl compound feed. The temperature at the reboiler was 177°C and the temperature at the condenser was 164°C. The pressure in the column was set at 100 psig (0.69 m Pa gauge). The boil up of water in the column was 500,000 pounds/hour (226,796 kg/hr). The conversion of EO was determined to be 99.99%, and the selectivity to ethylene glycol was 90.57%. Therefore, the "% Yield to MEG" was 90.57 for "Run 8". Runs 1 through 7 utilized equivalent parameters as Run 8.

**TABLE 1**

| Run# | # of Feed Ports | Location of Feed Ports | % Yield to MEG |
|---|---|---|---|
| 1 | 1 | 26 | 86.23 |
| 2 | 2 | 14, 26 | 88.64 |
| 3 | 3 | 8, 17, 26 | 89.49 |
| 4 | 4 | 8, 17, 23, 26 | 89.91 |
| 5 | 5 | 8, 14, 17, 23, 26 | 90.18 |
| 6 | 6 | 8, 11, 14, 17, 23, 26 | 90.35 |
| 7 | 7 | 8, 11, 14, 17, 20, 23, 26 | 90.48 |
| 8 | 8 | 5, 8, 11, 14, 17, 20, 23, 26 | 90.57 |

Other embodiments of the invention in addition to those exemplified above will be apparent to the skilled in the art from a consideration of this specification or practice of the invention disclosed herein. It is intended that the specification and example be considered as exemplary only, with the true scope of the invention being indicated by the following claims.

## Claims

1. A method for making glycol in an evaporation column reactor, the method comprising:
a) feeding a hydroxyl compound into the reactor;
b) feeding an epoxide compound into the reactor such that the epoxide compound is contacted with hydroxyl compound in a reaction zone under conditions such that substantially all of the epoxide compound reacts to form a product comprising a glycol which is dissolved in liquid hydroxyl compound;
c) removing the mixture of liquid hydroxyl compound and dissolved glycol away from the reaction zone;
d) heating the mixture of liquid hydroxyl compound and dissolved glycol such that at least some of the hydroxyl compound is evaporated away from the glycol; and
e) condensing and refluxing the evaporated hydroxyl compound and combining it with the hydroxyl compound of Step (a);
wherein the epoxide feed enters the reactor through at least two distinct ports within the reactor.

2. The method of Claim 1 wherein the evaporation column reactor comprises: a reaction zone for reacting the epoxide and hydroxyl compound; a reboiler zone which is located below the reaction zone; a condensing and refluxing zone which is located above the reaction zone; at least one hydroxyl feed port which is located above or within the reaction zone; and at least two epoxide feed ports which are located above the reboiler zone; wherein the reaction zone, reboiler zone, and condensing and reflux zone are all in fluid communication with each other.

3. The method of Claim 2 further comprising stripping any unreacted epoxide after Step (b) in a stripping section, wherein the stripping section is located within the reaction zone at an interface directly adjacent to the reboiler zone.

4. The method of Claim 3 wherein the stripping section comprises an inert packing material.

5. The method of Claim 2 wherein the reaction zone comprises a catalyst bed.

6. The method of Claim 5 wherein the catalyst bed comprises a catalyst selected from the group consisting essentially of aluminosilicate zeolites, amorphous aluminosilicates, and acid form ion exchange resins.

7. The method of Claim 5 wherein the catalyst bed comprises perfluorosulfonic acid resin entrapped within and dispersed throughout a carrier of metal oxide.

8. The method of Claim 5 wherein the catalyst bed comprises a catalytic grade wherein catalyst of highest activity is placed towards the top of the reaction zone and catalyst of lesser activity is placed towards the bottom of the reaction zone.

9. The method of any one of the preceding Claims wherein the epoxide is ethylene oxide.

10. The method of any one of the preceding Claims wherein the hydroxyl compound is selected from water, alcohol, and moieties of glycol.

11. The method of any one of the preceding Claims wherein the glycol product is selected from ethylene glycol, propylene glycol, and glycol ether.

12. The method of Claim 2 wherein three epoxide feed ports are utilized and different amounts of epoxide are fed into each epoxide feed port.

13. The method of Claim 2 further comprising enriching the evaporated hydroxyl compound in an enriching section by stripping out any undesired components wherein the enriching section is located within the reaction zone at an interface directly adjacent to the condensing and refluxing zone.

14. The method of any one of the preceding Claims wherein the method is conducted in at least two sequential effects of the evaporation column reactor.

15. A method for making glycol in a multiple effect evaporation column reactor, the method comprising:
a) feeding a hydroxyl compound into the reactor;
b) feeding an epoxide into the reactor such that the epoxide compound is contacted with hydroxyl compound in a reaction zone under conditions such that substantially all of the epoxide reacts to form a product comprising a glycol which is dissolved in liquid hydroxyl compound;
c) removing the mixture of liquid hydroxyl compound and dissolved glycol away from the reaction zone;
d) heating the mixture of liquid hydroxyl compound and dissolved glycol such that at least some of the hydroxyl compound is evaporated away from the glycol;
e) condensing and refluxing the evaporated hydroxyl compound and combining it with the hydroxyl compound of Step (a); and
f) transferring unevaporated hydroxyl compound, and glycol dissolved therein, from Step (d) to a subsequent effect of the evaporation column reactor at a point below the reaction zone of the subsequent effect such that it becomes the feed of the subsequent effect's Step (a);
wherein the transferring in Step (f) is continued for a desired number of times, with the final transfer being for the purpose of product recovery by transferring the unevaporated hydroxyl compound, and glycol dissolved therein, out of the multiple effect evaporation column reactor.

16. The method of Claim 15 wherein the epoxide feed of Step (b) enters at least one effect of the multiple effect evaporation column reactor through at least two distinct ports.

17. The method of Claim 15 wherein the mixture of liquid hydroxyl compound and dissolved glycol is heated by a reboiler in Step (d).

18. The method of Claim 15 wherein each subsequent effect of the multiple effect evaporation column reactor is maintained at a lower pressure than the pressure of its previous effect of the multiple effect evaporation column reactor.

19. The method of Claim 15 wherein the condensing and refluxing in Step (e) occurs in a condensing and refluxing zone which is located between one effect's reaction zone and another effect's reboiler zone such that, during condensation and reflux, heat is transferred from the evaporated hydroxyl compound to the reboiler zone of the other effect.

20. The method of Claim 15 wherein each subsequent evaporation column effect in series has a reaction zone of higher catalytic activity than the previous effect in series.

## Patentansprüche

1. Verfahren zur Herstellung von Glycol in einem Verdampfungskolonnenreaktor, wobei das Verfahren umfaßt:
a) Einspeisen einer Hydroxylverbindung in den Reaktor;
b) Einspeisen einer Epoxidverbindung in den Reaktor derart, daß die Epoxidverbindung mit der Hydroxylverbindung in einer Reaktionszone unter solchen Bedingungen in Kontakt gebracht wird, daß im wesentlichen alles der Epoxidverbindung unter Bildung eines Produktes reagiert, das ein Glycol umfaßt, welches in flüssiger Hydroxylverbindung aufgelöst ist;
c) Entfernen des Gemisches von flüssiger Hydroxylverbindung und aufgelöstem Glycol aus der Reaktionszone;
d) Erhitzen des Gemisches von flüssiger Hydroxylverbindung und ausgelöstem Glycol derart, daß wenigstens einiges der Hydroxylverbindung von dem Glycol wegverdampft wird;
e) Kondensieren und Rückflußkochen der verdampften Hydroxylverbindung und ihr Kombinieren mit der Hydroxylverbindung von Stufe (a);
bei welchem die Epoxideinspeisung in den Reaktor durch wenigstens zwei verschiedene Öffnungen in dem Reaktor eintritt.

2. Verfahren nach Anspruch 1, bei welchem der Verdampfungskolonnenreaktor umfaßt: eine Reaktionszone zur Umsetzung der Epoxid- und Hydroxylverbindung; eine Aufkochzone, welche unter der Reaktionszone angeordnet ist; eine Kondensier- und Rückflußzone, welche über der Reaktionszone angeordnet ist; wenigstens eine Hydroxyleinspeisungsöffnung, welche über oder innerhalb der Reaktionszone angeordnet ist; und wenigstens zwei Epoxideinspeisungsöffnungen, welche über der Aufkochzone angeordnet sind; wobei die Reaktionszone, Aufkochzone, Kondensier- und Rückflußzone alle in Fluidverbindung miteinander sind.

3. Verfahren nach Anspruch 2, weiter umfassend das Abstreifen von irgendwelchem nicht umgesetztem Epoxid nach Stufe (b) in einem Abstreifabschnitt, wobei der Abstreifabschnitt innerhalb der Reaktionszone an einer Grenzfläche direkt benachbart zur Aufkochzone angeordnet ist.

4. Verfahren nach Anspruch 3, bei welchem der Abstreifabschnitt ein inertes Packungsmaterial umfaßt.

5. Verfahren nach Anspruch 2, bei welchem die Reaktionszone ein Katalysatorbett umfaßt.

6. Verfahren nach Anspruch 5, bei welchem das Katalysatorbett einen Katalysator, ausgewählt aus der im wesentlichen aus Aluminosilikatzeolithen, amorphen Aluminosilikaten und Ionenaustauscherharzen in der Säureform bestehenden Gruppe, umfaßt.

7. Verfahren nach Anspruch 5, bei welchem das Katalysatorbett Perfluorsulfonsäureharz, eingeschlossen in und vollkommen dispergiert in einem Träger von Metalloxid, umfaßt.

8. Verfahren nach Anspruch 5, bei welchem das Katalysatorbett eine katalytische Abstufung umfaßt, wobei Katalysator höchster Aktivität nach dem Oberteil der Reaktionszone angeordnet ist und Katalysator geringerer Aktivität nach dem Unterteil der Reaktionszone angeordnet ist.

9. Verfahren nach einem der vorhergehenden Ansprüche, bei welchem das Epoxid Ethylenoxid ist.

10. Verfahren nach einem der vorhergehenden Ansprüche, bei welchem die Hydroxylverbindung aus gewählt ist aus Wasser, Alkohol und Glycoleinheiten.

11. Verfahren nach einem der vorhergehenden Ansprüche, bei welchem das Glycolprodukt ausgewählt ist aus Glycol, Propylenglycol und Glycolether.

12. Verfahren nach Anspruch 2, bei welchem drei Epoxideinspeisungsöffnungen angewandt werden und unterschiedliche Mengen von Epoxid in jede Epoxideinspeisungsöffnung eingespeist werden.

13. Verfahren nach Anspruch 2, weiter umfassend die Anreicherung der verdampften Hydroxylverbindung in einem Anreicherungsabschnitt durch Herausstreifen irgendwelcher unerwünschter Komponenten, wobei der Anreicherungsabschnitt innerhalb der Reaktionszone an einer Grenzfläche direkt benachbart zu der Kondensier- und Rückflußzone angeordnet ist.

14. Verfahren nach einem der vorhergehenden Ansprüche, bei welchem das Verfahren in wenigstens zwei aufeinanderfolgenden Einwirkstufen des Verdampfungskolonnenreaktors durchgeführt wird.

15. Verfahren zur Herstellung von Glycol in einem Mehrfacheffekt-Verdampfungskolonnenreaktor, wobei das Verfahren umfaßt:
a) Einspeisen einer Hydroxylverbindung in den Reaktor;
b) Einspeisen einer Epoxidverbindung in den Reaktor derart, daß die Epoxidverbindung mit der Hydroxylverbindung in einer Reaktionszone unter solchen Bedingungen in Kontakt gebracht wird, daß im wesentlichen alles des Epoxids unter Bildung eines Produktes reagiert, das ein Glycol umfaßt, welches in flüssiger Hydroxylverbindung aufgelöst ist;
c) Entfernen des Gemisches von flüssiger Hydroxylverbindung und aufgelöstem Glycol aus der Reaktionszone;
d) Erhitzen des Gemisches von flüssiger Hydroxylverbindung und aufgelöstem Glycol derart, daß wenigstens einiges der Hydroxylverbindung von dem Glycol wegverdampft wird;
e) Kondensieren und Rückflußkochen der verdampften Hydroxylverbindung und ihre Kombination mit der Hydroxylverbindung von Stufe (a); und
f) Überführen von nicht verdampfter Hydroxylverbindung und hierin aufgelöstem Glycol von Stufe (d) zu einer nachfolgenden Einwirkstufe des Verdampfungskolonnenreaktors bei einem Punkt unter der Reaktionszone der nachfolgenden Einwirkstufe, so daß sie die Einspeisung der Stufe (a) der nachfolgenden Einwirkstufe wird;
bei welchem die Überführung in Stufe (f) für eine gewünschte Anzahl von Malen fortgeführt wird, wobei die letzte Überführung zum Zweck der Produktgewinnung durch Überführung der nicht verdampften Hydroxylverbindung und hierin aufgelösten Glycols aus dem Mehrfacheffekt-Verdampfungskolonnenreaktor erfolgt.

16. Verfahren nach Anspruch 15, bei welchem die Epoxideinspeisung von Stufe (b) bei wenigstens einer Einwirkstufe des Mehrfacheffekt-Verdampfungskolonnenreaktors durch wenigstens zwei verschiedene Öffnungen eintritt.

17. Verfahren nach Anspruch 15, bei welchem das Gemisch aus flüssiger Hydroxylverbindung und aufgelöstem Glycol durch einen Aufkocher in Stufe (d) erhitzt wird.

18. Verfahren nach Anspruch 15, bei welchem jede nachfolgende Einwirkstufe des Mehrfacheffekt-Verdampfungskolonnenreaktor bei einem niedrigeren Druck als dem Druck ihrer vorangegangenen Einwirkstufe des Mehrfacheffekt-Verdampfungskolonnenreaktors gehalten wird.

19. Verfahren nach Anspruch 15, bei welchem das Kondensieren und Rückflußkochen in Stufe (e) in einer Kondensier- und Rückflußzone erfolgt, die zwischen der Reaktionszone einer Einwirkstufe und der Aufkochzone einer anderen Einwirkstufe angeordnet ist, derart, daß während Kondensieren und Rückfluß Wärme von der verdampften Hydroxylverbindung zu der Aufkochzone der anderen Einwirkstufe überführt wird.

20. Verfahren nach Anspruch 15, bei welchem jede nachfolgende Verdampfungskolonnen-Einwirkstufe in Reihe eine Reaktionszone von höherer katalytischer Aktivität als die vorangegangene Einwirkstufe in Reihe hat.

## Revendications

1. Procédé de préparation de glycols dans un réacteur de type colonne d'évaporation, ledit procédé comportant :
a) le fait d'amener dans le réacteur un composé hydroxylé ;
b) le fait d'amener dans le réacteur un époxyde, de façon à mettre cet époxyde en contact avec le composé hydroxylé, dans une zone de réaction, dans des conditions telles que pratiquement tout l'époxyde réagisse, pour obtenir un produit comportant un glycol dissous dans le composé hydroxylé liquide ;
c) le fait d'éloigner de la zone de réaction le mélange de composé hydroxylé liquide et de glycol dissous ;
d) le fait de chauffer ce mélange de composé hydroxylé liquide et de glycol dissous, de manière à ce qu'au moins une partie du composé hydroxylé s'évapore et soit ainsi séparée du glycol ; et
e) le fait de faire se condenser en reflux le composé hydroxylé évaporé et de le joindre au composé hydroxylé de l'étape (a) ;
dans lequel procédé l'époxyde amené dans le réacteur y pénètre par au moins deux orifices distincts.

2. Procédé conforme à la revendication 1, dans lequel le réacteur de type colonne d'évaporation comporte :
- une zone de réaction, où réagissent l'époxyde et le composé hydroxylé ;
- une zone servant de rebouilleur, située au-dessous de la zone de réaction ;
- une zone de condensation et reflux, située au-dessus de la zone de réaction ;
- au moins un orifice d'alimentation en composé hydroxylé, situé au-dessus de la zone de réaction ou au niveau de celle-ci ;
- et au moins deux orifices d'alimentation en époxyde, situés au-dessus du rebouilleur ;
la zone de réaction, le rebouilleur et la zone de condensation et reflux étant toutes en communication de fluide les unes avec les autres.

3. Procédé conforme à la revendication 2, qui comporte en outre le fait d'éliminer par stripage, après l'étape (b), l'époxyde n'ayant pas réagi, dans une section de stripage qui est située dans la zone de réaction, au niveau de l'interface de contact direct avec la zone servant de rebouilleur.

4. Procédé conforme à la revendication 3, dans lequel la section de stripage contient un garnissage en matériau inerte.

5. Procédé conforme à la revendication 2, dans lequel la zone de réaction contient un lit de catalyseur.

6. Procédé conforme à la revendication 5, dans lequel le lit de catalyseur contient un catalyseur choisi dans l'ensemble essentiellement constitué des zéolites de type aluminosilicate, des aluminosilicates amorphes, et des résines échangeuses d'ions sous forme acide.

7. Procédé conforme à la revendication 5, dans lequel le lit de catalyseur contient une résine acide perfluoro-sulfonique piégée au sein d'un support en oxyde de métal et dispersée dans tout ce support.

8. Procédé conforme à la revendication 5, dans lequel le lit de catalyseur présente un gradient catalytique, avec du catalyseur d'activité relativement forte en haut de la zone de réaction et du catalyseur d'activité relativement faible en bas de la zone de réaction.

9. Procédé conforme à l'une des revendications précédentes, dans lequel l'époxyde est de l'oxyde d'éthylène.

10. Procédé conforme à l'une des revendications précédentes, dans lequel le composé hydroxylé est choisi parmi l'eau, les alcools et les glycols.

11. Procédé conforme à l'une des revendications précédentes, dans lequel le glycol produit est choisi parmi l'éthylèneglycol, le propylèneglycol et les éthers de glycol.

12. Procédé conforme à la revendication 2, dans lequel on se sert de trois orifices d'alimentation en époxyde, en amenant des quantités différentes d'époxyde par chacun de ces orifices.

13. Procédé conforme à la revendication 2, qui comporte en outre le fait d'enrichir le composé hydroxylé évaporé en en éliminant par stripage tout composant indésirable, dans une section d'enrichissement qui est située dans la zone de réaction, au niveau de l'interface de contact direct avec la zone de condensation et reflux.

14. Procédé conforme à l'une des revendications précédentes, qui se déroule dans au moins deux secteurs successifs du réacteur de type colonne d'évaporation.

15. Procédé de préparation de glycols dans un réacteur de type colonne d'évaporation à secteurs multiples, ledit procédé comportant :
a) le fait d'amener dans le réacteur un composé hydroxylé ;
b) le fait d'amener dans le réacteur un époxyde, de façon à mettre cet époxyde en contact avec le composé hydroxylé, dans une zone de réaction, dans des conditions telles que pratiquement tout l'époxyde réagisse, pour obtenir un produit comportant un glycol dissous dans le composé hydroxylé liquide ;
c) le fait d'éloigner de la zone de réaction le mélange de composé hydroxylé liquide et de glycol dissous ;
d) le fait de chauffer ce mélange de composé hydroxylé liquide et de glycol dissous, de manière à ce qu'au moins une partie du composé hydroxylé s'évapore et soit ainsi séparée du glycol ;
e) le fait de faire se condenser en reflux le composé hydroxylé évaporé et de le joindre au composé hydroxylé de l'étape (a) ; et
f) le fait de faire passer le composé hydroxylé non évaporé lors de l'étape (d), avec le glycol dissous dedans, dans le secteur suivant du réacteur de type colonne d'évaporation, en un point situé au-dessous de la zone de réaction de ce secteur suivant, de façon qu'il constitue l'alimentation de l'étape (a) du secteur suivant ;
dans lequel procédé le transfert de l'étape (f) est répété autant de fois qu'on le veut, l'opération finale de transfert ayant pour but de récupérer le produit en faisant passer le composé hydroxylé non évaporé, avec le glycol dissous dedans, hors du réacteur de type colonne d'évaporation à secteurs multiples.

16. Procédé conforme à la revendication 15, dans lequel l'époxyde amené lors de l'étape (b) pénètre par au moins deux orifices distincts dans au moins l'un des secteurs du réacteur de type colonne d'évaporation à secteurs multiples.

17. Procédé conforme à la revendication 15, dans lequel, lors de l'étape (d), le mélange de composé hydroxylé liquide et de glycol dissous est chauffé au moyen d'un rebouilleur.

18. Procédé conforme à la revendication 15, dans lequel on maintient, dans chacun des secteurs successifs du réacteur de type colonne d'évaporation à secteurs multiples, une pression plus faible que dans le secteur précédent.

19. Procédé conforme à la revendication 15, dans lequel l'étape (e) de condensation en reflux se déroule dans une zone de condensation et reflux qui est située entre la zone de réaction d'un secteur et la zone rebouilleur d'un autre secteur, de sorte que, pendant la condensation en reflux, de la chaleur passe du composé hydroxylé évaporé à la zone rebouilleur de l'autre secteur.

20. Procédé conforme à la revendication 15, dans lequel, dans chacun des secteurs successifs de la colonne d'évaporation, l'activité catalytique dans la zone de réaction est plus forte que dans le secteur précédent de la série.
